# EUROPÄISCHE PATENTSCHRIFT

(11) **EP 1 173 521 B1**
(45) Veröffentlichungstag und Bekanntmachung des Hinweises auf die Patenterteilung: **16.10.2002**
(21) Anmeldenummer: 00926970.5
(22) Anmeldetag: 17.04.2000
(51) Int. Cl.: C09B 61/00, A23L 1/275

(54) **STABILE, WASSRIGE DISPERSIONEN UND STABILE, WASSERDISPERGIERBARE TROCKENPULVER VON XANTHOPHYLLEN, DEREN HERSTELLUNG UND VERWENDUNG**
STABLE, AQUEOUS DISPERSIONS AND STABLE, WATER-DISPERSIBLE DRY POWDERS OF XANTHOPHYLLS, AND PRODUCTION AND USE OF THE SAME
DISPERSIONS AQUEUSES STABLES ET POUDRES SECHES STABLES DISPERSIBLES DANS L'EAU DE XANTHOPHYLLES, LEUR PREPARATION ET LEUR UTILISATION

(30) Priorität: 29.04.1999 DE 19919751
(43) Veröffentlichungstag der Anmeldung: 23.01.2002
(73) Patentinhaber: BASF AKTIENGESELLSCHAFT, 67056 Ludwigshafen (DE)
(72) Erfinder: AUWETER, Helmut, D-67117 Limburgerhof (DE); BOHN, Heribert, D-67319 Wattenheim (DE); LÜDDECKE, Erik, D-67112 Mutterstadt (DE)
(86) Internationale Anmeldenummer: EP0003467
(87) Internationale Veröffentlichungsnummer: WO00066665

(56) Entgegenhaltungen:
- EP-A- 0 065 193
- EP-A- 0 278 284
- DE-A- 19 651 681
- GB-A- 918 399

## Beschreibung

Die Erfindung betrifft stabile, wäßrige Dispersionen und stabile, wasserdispergierbare Trockenpulver von Xanthophyllen, ausgewählt aus der Gruppe, bestehend aus Astaxanthin, Lutein und Zeaxanthin, deren Herstellung und Verwendung.

Die Stoffklasse der Carotinoide klassifiziert man in zwei Hauptgruppen, die Carotine und die Xanthophylle. Im Unterschied zu den Carotinen, bei denen es sich um reine Polyen-Kohlenwasserstoffe handelt, wie beispielsweise β-Carotin oder Lycopin, kommen in den Xanthophyllen noch Sauerstoff-Funktionen wie Hydroxy-, Epoxyund/oder Oxogruppen vor. Typische Vertreter dieser Gruppe sind u.a. Astaxanthin, Lutein und Zeaxanthin.

Xanthophylle sind in der Natur weit verbreitet und kommen u.a. im Mais (Zeaxanthin), in grünen Bohnen (Lutein), in Paprika (Capsanthin), in Eidottern (Lutein) sowie in Krebsen und Lachsen (Astaxanthin) vor, wobei sie diesen Nahrungsmitteln ihre charakteristische Färbung verleihen.

Diese sowohl technisch herstellbaren als auch aus natürlichen Quellen isolierbaren Polyene stellen für die Lebens- und Futtermittelindustrie und für den pharmazeutischen Bereich wichtige Farbkörper dar und sind, wie im Falle von Astaxanthin, Wirkstoffe mit Provitamin-A Aktivität.

Alle Xanthophylle sind in Wasser unlöslich, während in Fetten und Ölen eine jedoch nur geringe Löslichkeit gefunden wird. Diese begrenzte Löslichkeit sowie die hohe Oxidationsempfindlichkeit stehen einer direkten Anwendung der relativ grobkörnigen bei der Synthese erhaltenen Produkte in der Einfärbung von Lebens- und Futtermitteln entgegen, da die Substanzen in grobkristalliner Form nur schlechte Färbungsergebnisse liefern. Diese für die praktische Verwendung der Xanthophylle nachteiligen Effekte wirken sich insbesondere im wäßrigen Medium aus.

Nur durch gezielt hergestellte Formulierungen, in denen die Wirkstoffe in fein verteilter Form und gegebenenfalls durch Schutzkolloide oxidationsgeschützt vorliegen, lassen sich bei der direkten Einfärbung von Lebensmitteln verbesserte Farbausbeuten erzielen. Außerdem führen diese in Futtermitteln verwendeten Formulierungen zu einer höheren Bioverfügbarkeit der Xanthophylle und damit indirekt zu besseren Färbungseffekten z.B. bei der Eidotter- oder Fischpigmentierung.

Zur Verbesserung der Farbausbeuten und zur Erhöhung der Resorbierbarkeit bzw. Bioverfügbarkeit sind verschiedene Verfahren beschrieben worden, die alle das Ziel haben, die Kristallitgröße der Wirkstoffe zu verkleinern und auf einen Teilchengrößenbereich von kleiner 10 µm zu bringen.

Zahlreiche Methoden, u.a. beschrieben in Chimia 21, 329 (1967), WO 91/06292 sowie in WO 94/19411, bedienen sich dabei der Vermahlung von Carotinoiden mittels einer Kolloidmühle und erzielen damit Partikelgrößen von 2 bis 10 µm.

Daneben existieren eine Reihe von kombinierten Emulgier-/Sprühtrocknungsverfahren, wie sie z.B. in DE-A-12 11 911 oder in EP-A-0 410 236 beschrieben sind.

Gemäß der europäischen Patentschrift EP-B-0 065 193 erfolgt die Herstellung von feinverteilten, pulverförmigen β-Carotinpräparaten dadurch, daß man β-Carotin in einem flüchtigen, mit Wasser mischbaren organischen Lösungsmittel bei Temperaturen zwischen 50°C und 200°C, gegebenenfalls unter erhöhtem Druck, innerhalb einer Zeit von weniger als 10 Sekunden löst. Aus der erhaltenen molekulardispersen Lösung wird das β-Carotin durch sofortiges schnelles Mischen mit einer wäßrigen Lösung eines Schutzkolloids bei Temperaturen zwischen 0°C und 50°C ausgefällt. Man erhält so ein kolloid-disperses β-Carotin-Hydrosol mit orange-gelber Farbnuance. Anschließende Sprühtrocknung der Dispersion liefert ein freifließendes Trockenpulver, das sich in Wasser unter Bildung einer klaren, gelborange gefärbten Dispersion löst.

Bei den nach EP-B-0 065 193 hergestellten nanopartikulären Wirkstoffdispersionen von Xanthophyllen sind jedoch folgende Phänomene zu beobachten.

Die wäßrigen, Xanthophyll-haltigen Wirkstoffdispersionen sind häufig, insbesondere bei der Aufkonzentration, kolloidal instabil. Durch Ausflockungen der Wirkstoffpartikel, die dabei teilweise sedimentieren, teilweise aufrahmen, ist eine weitere Überführung der Dispersion in ein Trockenpulver nicht mehr möglich.

Bei Xanthophyllen mit Carbonyl-Funktionen kann außerdem die als alleiniges Schutzkolloid eingesetzte Gelatine vernetzen, so daß ein Gel entsteht, das nicht mehr redispergierbar ist und das ebenfalls nicht weiter in ein Trockenpulver überführt werden kann.

Somit können die hohen Anforderungen an Xanthophyll-haltigen Formulierungen bezüglich Farbwirkung und Bioverfügbarkeit aufgrund der geschilderten Problematik mit dem o.g. Verfahren nicht immer erfüllt werden.

Wie in WO 98/26008 beschrieben, läßt sich durch Verwendung eines Gemisches aus niedermolekularen und hochmolekularen Schutzkolloiden die Redispergierbarkeit der Xanthophyll-haltigen Trockenpulver verbessern.

Es bestand daher die Aufgabe, ein Verfahren zur Herstellung einer stabilen, wässrigen Dispersion von Xanthophyllen, ausgewählt aus der Gruppe, bestehend aus Astaxanthin, Lutein und Zeaxanthin vorzuschlagen. Ferner sollten stabile, pulverförmige Zubereitungen der o.g. Xanthophylle zur Verfügung gestellt werden, mit denen eine gute Farbwirkung und zudem eine hohe Bioverfügbarkeit erzielt werden können.

Diese Aufgabe wurde erfindungsgemäß gelöst mit einem Verfahren zur Herstellung einer stabilen, wäßrigen Dispersion oder eines stabilen wasserdispergierbaren Trockenpulvers von Xanthophyllen, ausgewählt aus der Gruppe, bestehend aus Astaxanthin, Lutein und Zeaxanthin durch
a) Lösen eines oder mehrerer der Xanthophylle, ausgewählt aus der Gruppe, bestehend aus Astaxanthin, Lutein und Zeaxanthin, in einem mit Wasser mischbaren, organischen Lösungsmittel oder einer Mischung aus Wasser und einem mit Wasser mischbaren, organischen Lösungsmittel bei Temperaturen größer 30°C,
b) Mischen dieser Lösung mit einer wäßrigen Lösung eines Schutzkolloids, wobei die Lösungsmittelkomponente in die wäßrige Phase überführt wird und die hydrophobe Phase des Xanthophylls als nanodisperse Phase entsteht
c) und gegebenenfalls Überführung der gebildeten Dispersion in ein wasserdispergierbares Trockenpulvers durch Abtrennung des Lösungsmittels und des Wassers und Trocknung, gegebenenfalls in Gegenwart eines Überzugsmaterials,
dadurch gekennzeichnet, daß man als Schutzkolloid im Verfahrensschritt b) Casein oder ein Caseinat verwendet.

Die Herstellung der erfindungsgemäßen Zubereitungen erfolgt in der Regel so, daß man mindestens eines der o.g. Xanthophylle, gegebenenfalls zusammen mit einem eßbaren Öl, in einem mit Wasser mischbaren, organischen Lösungsmittel bei Temperaturen größer 30°C, vorzugsweise zwischen 50°C und 240°C, insbesondere 100°C bis 200°C, besonders bevorzugt 140°C bis 180°C, gegebenenfalls unter Druck, löst.

Da die Einwirkung hoher Temperaturen den gewünschten hohen alltrans Isomerenanteil herabsetzen kann, löst man das/die Xanthophyll(e) möglichst rasch, beispielsweise im Sekundenbereich, z.B. in 0,1 bis 10 Sekunden, besonders bevorzugt in weniger als 1 Sekunde. Zur raschen Herstellung der molekulardispersen Lösung kann die Anwendung von erhöhtem Druck, z.B. im Bereich von 20 bar bis 80 bar, vorzugsweise 30 bis 60 bar, vorteilhaft sein.

Die so erhaltene molekulardisperse Lösung versetzt man unmittelbar anschließend mit der gegebenenfalls gekühlten wäßrigen Lösung des Caseins oder Caseinats bevorzugt in der Weise, daß sich eine Mischungstemperatur von etwa 35°C bis 80°C einstellt.

Dabei wird die Lösungsmittelkomponente in die wäßrige Phase überführt und die hydrophobe Phase des/der Xanthophyll(e) entsteht als nanodisperse Phase.

Hinsichtlich einer näheren Verfahrens- und Apparatebeschreibung wird an dieser Stelle ausdrücklich auf EP-B-0 065 193 Bezug genommen.

Als Schutzkolloide werden nieder- und/oder hochmolekulares Kasein oder Caseinat oder Mischungen davon verwendet. Bevorzugt verwendet man Na-Caseinat mit einem Molekulargewicht von 10000 bis 100000, besonders bevorzugt mit einem MW von 20000 bis 60000, beispielsweise Na-Caseinat der Fa. Lacto Bretagne Associés S.A. (Frankreich) mit einem MW von ca. 38000.

Einzelheiten zum eingesetzten Casein/Caseinat finden sich u.a. in Ullmann's Encyclopedia of Industrial Chemistry, 6^{th} Edition, 1998 Electronic Release, Chapter 11.1., Wiley-VCH, Weinheim, Germany sowie in CD Römpp Chemie Lexikon-Version 1.0, Stuttgart/New York: Georg Thieme Verlag 1995 und der darin zitierten Literatur.

Zur Erhöhung der mechanischen Stabilität des Endproduktes ist es zweckmäßig, dem Kolloid einen Weichmacher zuzusetzen, wie Zucker oder Zuckeralkohole, z.B. Saccharose, Glucose, Lactose, Invertzucker, Sorbit, Mannit oder Glycerin.

Das Verhältnis Schutzkolloid und Weichmacher zu Xanthophyll-Lösung wird im allgemeinen so gewählt, daß ein Endprodukt erhalten wird, das zwischen 0,5 und 30 Gew.-%, bevorzugt 5 bis 25 Gew.-%, besonders bevorzugt 10 bis 23 Gew.-% Xanthophyll, 10 bis 70 Gew.-% eines Schutzkolloids, 10 bis 70 Gew.-% eines Weichmachers, alle Prozentangaben bezogen auf die Trockenmasse des Pulvers, sowie gegebenenfalls geringe Mengen eines Stabilisators enthält.

Die Xanthophylle, die bei der Durchführung der Erfindung eingesetzt werden können, sind die bekannten, natürlichen oder synthetischen zugänglichen Verbindungen Astaxanthin, Lutein und/oder Zeaxanthin. Als bevorzugt eingesetztes Xanthophyll für das erfindungsgemäße Verfahren ist Astaxanthin zu nennen.

Zur Erhöhung der Stabilität des Wirkstoffes gegen oxidativen Abbau ist es vorteilhaft, Stabilisatoren wie α-Tocopherol, t-Butylhydroxy-toluol, t-Butylhydroxyanisol, Ascorbinsäure oder Ethoxyquin zuzusetzen. Sie können entweder der wäßrigen oder der Lösungsmittel-Phase zugesetzt werden, vorzugsweise werden sie jedoch gemeinsam mit den Xanthophyllen in der Lösungsmittel-Phase gelöst.

Unter Umständen kann es auch vorteilhaft sein, zusätzlich in der Lösungsmittel-Phase ein physiologisch zugelassenes Öl wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.-%, vorzugsweise 10 bis 300 Gew.-%, besonders bevorzugt 20 bis 100 Gew.-%, bezogen auf das/die Xanthophyll(e), zu lösen, das dann gemeinsam mit den Wirkstoffen und den genannten Zusatzstoffen beim Mischen mit der wäßrigen Phase extrem feinteilig ausgefällt wird.

Überraschenderweise kann bei der erfindungsgemäßen Verwendung von Casein oder Caseinat als Schutzkolloid auf den Einsatz zusätzlicher Emulgatoren, wie z.B. Ascorbylpalmitat verzichtet werden.

Darüberhinaus wurde gefunden, daß man kolloidal stabile und nicht vernetzende nanopartikuläre Wirkstoffdispersionen von Xanthophyllen erhält, deren Viskositätsverhalten annähernd denen von Newtonschen Flüssigkeiten entspricht. Derartige Flüssigkeiten zeichnen sich dadurch aus, daß deren durch die Newtonsche Gleichung τ = h·D definierter Fließwiderstand bei gegebener Temperatur eine Stoffkonstante ist (τ = Schubspannung, D = Schergefälle, h = dynamische Viskosität). Die graphische Darstellung des Fließverhaltens newtonscher Flüssigkeiten ergibt bei gegebener Temperatur annähernd eine Gerade. Insbesondere ändert sich die Viskosität der Wirkstoffdispersion bei 40°C und bei 60°C im Scherbereich zwischen 10⁻² sec⁻¹ und 10⁺² sec⁻¹ um weniger als ±50 %.

Die Vorteile dieses annähernd newtonschen Viskositätsverhaltens liegen u.a. darin, daß sich die Wirkstoffdispersionen, insbesondere nach der Aufkonzentrierung leichter Pumpen lassen, als dies bei strukturviskosen Dispersionen der Fall ist. Beim Sprühtrocknen haben die annähernd newtonschen Wirkstoffdispersionen außerdem den Vorteil, daß sich die Parameter des Sprühkopfes leichter optimieren lassen und daß sich diese Dispersionen im Sprühkopf unkritischer verhalten.

Ferner konnte beobachtet werden, daß mit dem Verfahren die Zusammenlagerung der Xanthophylle zu H-Aggregaten vermieden wird.

Die Aggregation von Carotinoiden ist ein in der Literatur bereits bekanntes und in zahlreichen Publikationen beschriebenes Phänomen [P. Song, T.A. Moore, Photochemistry and Photobiology, 19, 435-441 (1974); A.V. Ruban, P. Horton, A.J. Young, J. Photochem. Photobiol. B: Biol., 21, 229-234 (1993); V.R. Salares, N.M. Young, P.R. Carey, H.J. Bernstein, Journal of Raman Spectroscopy, 6(6), 282-288 (1977)].

Carotinoid-Aggregate können beispielsweise dadurch erzeugt werden, daß man eine Lösung eines Carotinoids in einem wassermischbaren, organischen Lösungsmittel wie z.B. Isopropanol, Ethanol, Aceton oder Tetrahydrofuran mit Wasser vermischt.

So können, wie in der oben genannten Literatur beschrieben, bei Wahl der richtigen Mengenverhältnisse von Wasser und organischem Lösungsmittel entweder sogenannte H- oder J-Aggregate erzeugt werden.

Unter H-Aggregaten versteht man eine "kartenspielähnliche" Stapelung der Polyenketten (card-stack aggregate), die sich im UV/Vis-Spektrum durch das Auftreten einer neuen, im Vergleich zur Absorption der monomer vorliegenden Formen hypsochrom verschobenen Bande im Bereich zwischen 320 und 400 nm charakterisieren läßt. J-Aggregate dagegen stellen entweder eine lineare Kopf-Schwanz Verknüpfung (head-tail aggregates) der Polyene dar oder sie sind fischgrätenartig angeordnet (herringbone aggregates). Beide Anordnungen bewirken eine bathochrome Verschiebung der UV-Absorption der Polyene.

Fütterungsversuche an Forellen haben gezeigt, daß H-Aggregate von Xanthophyllen, insbesondere die H-Aggregate von Astaxanthin eine schlechtere Bioverfügbarkeit aufweisen als die entsprechenden J-Aggregate, was sich als ein weiterer Vorteil der nach dem erfindungsgemäßen Verfahren hergestellten Dispersionen bzw. Trockenpulver darstellt.

Je nach Menge des verwendeten Caseins oder Caseinats erhält man eine tiefgefärbte viskose Flüssigkeit. Die Entfernung des Lösungsmittels kann beispielsweise durch Extraktion mit einem mit Wasser nicht mischbaren Lösungsmittel oder, je nach Siedepunkt in an sich bekannter Weise, z.B. durch Destillation, gegebenenfalls unter vermindertem Druck, erfolgen. In diesem Fall hat es sich als zweckmäßig und möglich erwiesen, das bei Verwendung von Isopropanol erhaltene Azeotrop ohne Wasserentfernung unmittelbar als Lösungsmittel einzusetzen. Vorzugsweise erfolgt die Lösungsmittelabtrennung jedoch gemeinsam mit der Entfernung des Wassers durch Sprühtrocknung oder Sprühgranulation.

Die Erfindung betrifft somit auch stabile, wäßrige Dispersionen sowie stabile wasserdispergierbare Trockenpulver von Xanthophyllen, aus der Gruppe, bestehend aus Astaxanthin, Lutein und Zeaxanthin, die nach dem oben beschriebenen Verfahren erhältlich sind.

Man erhält ein stabiles Trockenpulver, das dadurch gekennzeichnet ist, daß es von einem Casein, Caseinat oder Mischungen davon als Schutzkolloid umhüllt ist. Dieses Trockenpulver kann erneut in Wasser unter Erzielung einer gleichmäßigen Feinverteilung des Wirkstoffes im Korngrößenbereich kleiner 1 µm gelöst werden. Im photochemischen Stabilitätstest erweist sich das so erhaltene Wirkstoff-Hydrosol trotz der Feinverteilung als außerordentlich stabil.

Sowohl in der wäßrigen Xanthophyll-Dispersion, als auch im daraus hergestellten Trockenpulver weist der enthaltene Wirkstoff einen, anhand von Röntgenbeugungsdiagrammen ermittelten, amorphen Anteil zwischen 70 und 100 %, bevorzugt zwischen 90 und 100 % auf. Ferner beträgt der all-trans Isomerengehalt der Xanthophylle mindestens 50 %, bevorzugt 70 %.

Die erfindungsgemäßen Zubereitungen eignen sich hervorragend als Lebens- und Futtermittelfarbstoff sowie als Zusatz zu Pharmazeutika. Typische Einsatzgebiete im Futtermittelbereich sind beispielsweise die Fischpigmentierung in der Aquakultur sowie die Eidotter- und Broilerhautpigmentierung in der Geflügelzucht.

In dem nachfolgenden Beispiel wird die Durchführung des erfindungsgemäßen Verfahrens näher erläutert.

### Beispiel 1

In einer beheizbaren Vorlage wurden bei einer Temperatur von 30°C 40 g Astaxanthin in 294 g Isopropanol/Wasser (88/12, w/w) suspendiert. Diese Suspension wurde in einer Mischkammer bei einer Mischungstemperatur von 170°C mit 536 g Isopropanol/Wasser (88/12, w/w) bei einer Verweilzeit von 0,2 Sekunden gemischt. Nach der genannten Verweilzeit trat die entstandene molekulardisperse Astaxanthin-Lösung unmittelbar anschließend in eine weitere Mischkammer ein, in der unter einem Mischungswinkel von 90° über eine Hochdruckpumpe 10,4 kg einer auf pH 9 eingestellten, wäßrigen Na-Caseinatlösung, die neben 108 g Caseinat 36 g Saccharose enthielt, zugemischt wurden, wobei das Astaxanthin in kolloiddisperser Form mit einer mittleren Teilchengröße von 144 nm bei einer Temperatur von 45°C ausfiel.

Anschließend wurde die Dispersion aufkonzentriert und in an sich bekannter Weise in ein freifließendes 22%iges Astaxanthin-Trockenpulver mit einer mittleren Teilchengröße von 129 nm überführt. Das Trockenpulver löste sich in Wasser wiederum unter Bildung einer klaren, rot gefärbten Dispersion, wobei die Farbstärke der Re-Dispersion lediglich um ca. 10 %, bezogen auf die ursprüngliche Dispersion, abnahm.

## Patentansprüche

1. Verfahren zur Herstellung einer stabilen, wäßrigen Dispersion oder eines stabilen wasserdispergierbaren Trockenpulvers von Xanthophyllen, ausgewählt aus der Gruppe, bestehend aus Astaxanthin, Lutein und Zeaxanthin, durch
a) Lösen eines oder mehrerer der Xanthophylle, ausgewählt aus der Gruppe, bestehend aus Astaxanthin, Lutein und Zeaxanthin, in einem mit Wasser mischbaren, organischen Lösungsmittel oder einer Mischung aus Wasser und einem mit Wasser mischbaren, organischen Lösungsmittel bei Temperaturen größer 30°C,
b) Mischen dieser Lösung mit einer wäßrigen Lösung eines Schutzkolloids, wobei die Lösungsmittelkomponente in die wäßrige Phase überführt wird und die hydrophobe Phase des Xanthophylls als nanodisperse Phase entsteht
c) und gegebenenfalls Überführung der gebildeten Dispersion in ein wasserdispergierbares Trockenpulvers durch Abtrennung des Lösungsmittels und des Wassers und Trocknung, gegebenenfalls in Gegenwart eines Überzugsmaterials,
**dadurch gekennzeichnet, daß** man als Schutzkolloid im Verfahrensschritt b) Casein oder ein Caseinat verwendet.

2. Verfahren nach Anspruch 1, **dadurch gekennzeichnet, daß** man im Verfahrensschritt a) zusätzlich zum Xanthophyll ein eßbares Öl verwendet.

3. Verfahren nach einem der Ansprüche 1 oder 2, **dadurch gekennzeichnet, daß** man keinen zusätzlichen Emulgator verwendet.

4. Verfahren nach einem der Ansprüche 1 bis 3, **dadurch gekennzeichnet, daß** man die Bildung von H-Aggregaten der Xanthophylle vermeidet.

5. Verfahren nach einem der Ansprüche 1 bis 4, **dadurch gekennzeichnet, daß** man als Xanthophyll Astaxanthin verwendet.

6. Stabile, wäßrige Dispersionen von Xanthophyllen, ausgewählt aus der Gruppe, bestehend aus Astaxanthin, Lutein und Zeaxanthin, erhältlich nach einem Verfahren, definiert gemäß einem der Ansprüche 1 bis 4.

7. Stabile, wäßrige Dispersionen von Xanthophyllen nach Anspruch 6, **dadurch gekennzeichnet, daß** das enthaltene Xanthophyll einen amorphen Anteil zwischen 70 und 100% aufweist.

8. Stabile, wäßrige Dispersionen von Xanthophyllen nach einem der Ansprüche 6 oder 7, **dadurch gekennzeichnet, daß** das enthaltene Xanthophyll einen all-trans Isomerengehalt von mindestens 50% aufweist.

9. Stabile, wäßrige Dispersionen von Xanthophyllen nach einem der Ansprüche 6 bis 8, **dadurch gekennzeichnet, daß** sie ein Viskositätsverhalten von Newtonschen Flüssigkeiten aufweisen.

10. Stabile, wäßrige Dispersionen von Xanthophyllen nach einem der Ansprüche 6 bis 9, **dadurch gekennzeichnet, daß** es sich um Dispersionen von Astaxanthin handelt.

11. Stabile, wasserdispergierbare Trockenpulver von Xanthophyllen, ausgewählt aus der Gruppe, bestehend aus Astaxanthin, Lutein und Zeaxanthin, erhältlich nach einem Verfahren, definiert gemäß einem der Ansprüche 1 bis 4.

12. Stabile, wasserdispergierbare Trockenpulver von Xanthophyllen, nach Anspruch 11, **dadurch gekennzeichnet, daß** es sich um Trockenpulver von Astaxanthin handelt.

13. Verwendung der stabilen, wäßrigen Dispersionen und/oder stabilen wasserdispergierbaren Trockenpulver von Xanthophyllen, definiert gemäß einem der Ansprüche 6 bis 12, als Zusatz zu Lebensmitteln, Pharmazeutika und/oder Tierfuttermitteln.

## Claims

1. A process for preparing a stable, aqueous dispersion or a stable water-dispersible dry powder of xanthophylls selected from the group consisting of astaxanthin, lutein and zeaxanthin, by
a) dissolving one or more of the xanthophylls selected from the group consisting of astaxanthin, lutein and zeaxanthin in a water-miscible organic solvent or a mixture of water and a water-miscible organic solvent at temperatures above 30°C,
b) mixing this solution with an aqueous solution of a protective colloid, the solvent component being transferred into the aqueous phase, and the hydrophobic phase of the xanthophyll resulting as nanodisperse phase
c) and, where appropriate, converting the dispersion which has formed into a water-dispersible dry powder by removing the solvent and the water and drying, where appropriate in the presence of a coating material,
wherein casein or a caseinate is used as protective colloid in step b).

2. A process as claimed in claim 1, wherein an edible oil is used in addition to the xanthophyll in step a).

3. A process as claimed in either of claims 1 or 2, wherein no additional emulsifier is used.

4. A process as claimed in any of claims 1 to 3, wherein the formation of H aggregates of the xanthophylls is avoided.

5. A process as claimed in any of claims 1 to 4, wherein astaxanthin is used as xanthophyll.

6. A stable aqueous dispersion of xanthophylls selected from the group consisting of astaxanthin, lutein and zeaxanthin, obtainable by a process as defined in any of claims 1 to 4.

7. A stable aqueous dispersion of xanthophylls as claimed in claim 6, wherein the xanthophyll present has an amorphous content between 70 and 100%.

8. A stable aqueous dispersion of xanthophylls as claimed in either of claims 6 or 7, wherein the xanthophyll present has an all-trans isomer content of at least 50%.

9. A stable aqueous dispersion of xanthophylls as claimed in any of claims 6 to 8, which shows the viscosity behavior of newtonian fluids.

10. A stable aqueous dispersion of xanthophylls as claimed in any of claims 6 to 9, which is a dispersion of astaxanthin.

11. A stable water-dispersible dry powder of xanthophylls selected from the group consisting of astaxanthin, lutein and zeaxanthin, obtainable by a process as defined in any of claims 1 to 4.

12. A stable water-dispersible dry powder of xanthophylls as claimed in claim 11, which is a dry powder of astaxanthin.

13. The use of the stable aqueous dispersions and/or stable water-dispersible dry powders of xanthophylls defined in any of claims 6 to 12 as addition to human foods, pharmaceuticals and/or animal feeds.

## Revendications

1. Procédé pour préparer une dispersion aqueuse stable ou une poudre sèche dispersable à l'eau, stable, de xanthophylles choisies dans le groupe consistant en l'astaxanthine, la lutéine et la zéaxanthine, par
a) dissolution d'une ou plusieurs des xanthophylles choisies dans le groupe consistant en l'astaxanthine, la lutéine et la zéaxanthine dans un solvant organique miscible à l'eau ou dans un mélange d'eau et d'un solvant organique miscible à l'eau à des températures supérieures à 30° C,
b) mélange de cette solution avec une solution aqueuse d'un colloïde protecteur, au cours duquel le composant solvant passe dans la phase aqueuse et la phase hydrophobe de la xanthophylle passe à l'état de phase nanodispersée,
c) et le cas échéant, conversion de la dispersion formée en une poudre sèche dispersable à l'eau, par séparation du solvant et de l'eau et séchage, éventuellement en présence d'un produit de revêtement,
**caractérisé par le fait que** l'on utilise en tant que colloïde protecteur au stade opératoire b) la caséine ou un caséinate.

2. Procédé selon revendication 1, **caractérisé par le fait que**, au stade opératoire a), en plus de la xanthophylle, on utilise une huile comestible.

3. Procédé selon l'une des revendications 1 ou 2, **caractérisé par le fait que** l'on n'utilise pas d'autre agent émulsionnant.

4. Procédé selon l'une des revendications 1 à 3, **caractérisé par le fait que** l'on évite la formation d'agglomérats H des xanthophylles.

5. Procédé selon l'une des revendications 1 à 4, **caractérisé par le fait que** la xanthophylle utilisée est l'astaxanthine.

6. Dispersions aqueuses stables de xanthophylles choisies dans le groupe consistant en l'astaxanthine, la lutéine et la zéaxanthine, obtenues par un procédé selon l'une des revendications 1 à 4.

7. Dispersions aqueuses stables de xanthophylles selon la revendication 6, **caractérisées par le fait que** la xanthophylle contenue dans la dispersion présente une fraction amorphe de 70 à 100 %.

8. Dispersions aqueuses stables de xanthophylles selon l'une des revendications 6 ou 7, **caractérisées en ce que** la xanthophylle contenue dans la dispersion présente une teneur d'au moins 50 % en isomère tout-trans.

9. Dispersions aqueuses stables de xanthophylles selon l'une des revendications 6 à 8, **caractérisées par le fait qu'**elles ont un comportement de viscosité de liquide newtonien.

10. Dispersions aqueuses stables de xanthophylles selon l'une des revendications 6 à 9, **caractérisées par le fait qu'**il s'agit de dispersions d'astaxanthine.

11. Poudre sèche, dispersable à l'eau et stable de xanthophylles choisies dans le groupe consistant en l'astaxanthine, la lutéine et la zéaxanthine, obtenue par un procédé selon l'une des revendications 1 à 4.

12. Poudre sèche, dispersable à l'eau et stable, de xanthophylle selon la revendication 11, **caractérisée par le fait qu'**il s'agit d'une poudre sèche d'astaxanthine.

13. Utilisation des dispersions aqueuses stables et/ou des poudres sèches dispersables à l'eau et stables de xanthophylles définies selon l'une des revendications 6 à 12, en tant qu'additifs à des produits alimentaires, des produits pharmaceutiques et/ou des aliments pour animaux.
